# EUROPÄISCHE PATENTSCHRIFT

(11) **EP 1 885 328 B1**
(45) Veröffentlichungstag und Bekanntmachung des Hinweises auf die Patenterteilung: **01.08.2012**
(21) Anmeldenummer: 06753516.1
(22) Anmeldetag: 09.05.2006
(51) Int. Cl.: A61K 8/44, A61K 8/49, A61K 8/55, A61K 8/60, A61Q 19/00, A61Q 7/00, A61Q 19/08, A61P 17/06, A61K 31/195, A61K 31/7032

(54) **WIRKSTOFFKOMBINATIONEN AUS GLUCOSYLGLYCERIDEN UND KREATIN UND/ODER KREATININ**
ACTIVE INGREDIENT COMBINATIONS OF GLUCOSYL GLYCERIDES AND CREATINE AND/OR CREATININE
COMBINAISONS DE MATIERES ACTIVES FORMEES DE GLYCERIDES DE GLUCOSYLE ET DE CREATINE ET/OU DE CREATININE

(30) Priorität: 19.05.2005 DE 102005023636
(43) Veröffentlichungstag der Anmeldung: 13.02.2008
(73) Patentinhaber: Beiersdorf AG, 20253 Hamburg (DE)
(72) Erfinder: BLATT, Thomas, 22880 Wedel (DE); BREITENBACH, Ute, 20251 Hamburg (DE); MUMMERT, Christopher, 29553 Bienenbüttel (DE); RASCHKE, Thomas, 25421 Pinneberg (DE); SCHERNER, Cathrin, 22455 Hamburg (DE); SCHULZ, Jens, 22869 Schenefeld (DE); STÄB, Franz, 21379 Echem (DE)
(86) Internationale Anmeldenummer: PCT/EP2006/004293
(87) Internationale Veröffentlichungsnummer: WO 2006/122668

(56) Entgegenhaltungen:
- EP-A- 0 770 378
- EP-A- 0 824 915
- DE-A1- 10 032 964
- DATABASE WPI Section Ch, Week 200458 Derwent Publications Ltd., London, GB; Class B04, AN 2004-597337 XP002395733 & JP 2004 229668 A (TATSUMA HONKE SHUZO KK) 19. August 2004 (2004-08-19)

## Beschreibung

Die vorliegende Erfindung betrifft Wirkstoffkombinationen aus Glucosylglyceriden und oberflächenaktiven Glucosederivaten sowie deren Verwendung auf dem Gebiete der kosmetischen sowie der pharmazeutischen Dermatologie.

Insbesondere betrifft die vorliegende Erfindung Wirkstoffe und kosmetische oder dermatologische Zubereitungen, solche Wirkstoffkombinationen enthaltend.

Insbesondere betrifft die vorliegende Erfindung kosmetische Zubereitungen mit einem Gehalt an Substanzen welche die Hautfeuchtigkeit steigern.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muß deshalb ununterbrochen erneuert werden.

Ein heute in der Fachwelt weitverbreitetes Hautmodell faßt das Stratum corneum als ZweiKomponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen. Hierzu zählen u.a. Polyole wie Glycerin, Sorbit und Xylit, ethoxylierte Polyole sowie hydrolysierte Proteine. Weitere Anwendung finden die im natürlichen Feuchthaltefaktor (sogenannter Natural Moisturizing Factor = NMF) enthaltenen Substanzen, wie z.B. Harnstoff, Kohlenhydrate (z. B. Glucose) und Aminosäuren (z. B. Serin). Diese Substanzen sind daher für die Pflegeleistung eines kosmetischen Produktes von besonderer Bedeutung, insbesondere auch aufgrund ihrer relativ guten Haut- und Schleimhautverträglichkeit.

Ungelöst ist jedoch das Problem, daß auch die grundsätzlich völlig unbedenklichen Substanzen Glucose und Glycerin in sehr hoher Dosierung bei besonders empfindlichen Personen bestimmte Haut- und Schleimhautreizungserscheinungen hervorrufen können. Ziel war es daher, feuchtigkeitsspendende Substanzen (sogenannte "Moisturizer") zu finden, die über eine noch bessere Verträglichkeit als beispielsweise Glucose und Glycerin verfügen.

Die vorteilhafte prophylaktische und therapeutische Wirkung des Kreatins bei der kosmetischen und medizinischen Hautpflege ist an sich bekannt. Kreatin (von grch.: τo κρεασ = "das Fleisch") zeichnet sich durch folgende Struktur aus:

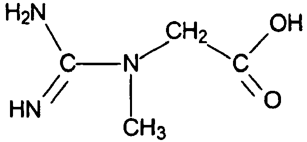

Es findet sich im Muskelsaft der Wirbeltiere zu 0,05-0,4%, in geringen Mengen auch im Gehirn und Blut. Als Monohydrat stellt es ein farbloses, kristallines Pulver dar. In wäßriger Lösung wird Kreatinin gebildet. Im Organismus entsteht es durch Transamidinierung von L-Arginin auf Glycin zu Guanidinoessigsäure und deren anschließende Methylierung mittels S-Adenosylmethionin (durch Guanidinoacetat-Methyltransferase). Man hält Kreatin für einen appetitfördernden Bestandteil von Rindfleisch und Fleischextrakt. Kreatinzusatz zur Nahrung verstärkt die körperliche Leistungsfähigkeit.

Umfangreich ist der Stand der Technik zu den kosmetischen und dermatologischen Verwendungen des Kreatins.

So beschreibt die DE 100 32 964 die Verwendung von Kreatin und/oder Kreatinderivaten in kosmetischen oder dermatologischen Zubereitungen zur Behandlung und Prophylaxe der Symptome von UV- und oder Ozon- induzierten Hautschäden sowie von entzündlichen und degenerativen Hautzuständen.

Die JP2000/247866 beschreibt Hautkosmetika mit einem Gehalt an Kreatin und/oder Kreatinin, welche als Creme oder als milchige Lotion verwendet werden können, wobei den betreffenden Zubereitungen vorzügliche hautpflegende Eigenschaften zugeschrieben werden.

Ferner beschreibt die WO00/33787 die Verwendung von Kreatin als wirksamen Bestandteil von Desodorantien.

Weiterhin beschreibt die EP-A 565 010 Haarwuchs- und Haarfärbezubereitungen mit einem Gehalt an Kreatinphosphat.

Schließlich werden in der US-A 4,590,067 und der EP-A-178 602 die Verwendung von Kreatin bzw. Kreatinin zur Herstellung von Zubereitungen mit antiinflammatorischer Wirksamkeit beschrieben.

Nachteilig ist jedoch, daß Kreatin in wasserhaltigen Produkten leicht auskristallisiert, wobei Kristalle mit unkosmetischer Anmutung entstehen und die Wirksamkeit des Produktes vermindert wird.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Eine weitere Aufgabe bestand darin, eine Darreichungsform für Kreatin zu finden, die sich durch verminderte Neigung zur Bildung von Kreatinkristallen auszeichnet.

Es war nach all diesem überraschend und nicht vorhersehbar, daß Wirkstoffkombinationen aus
(i) einem oder mehreren Glucosylglyceriden gewählt aus den Substanzen der allgemeinen Formel 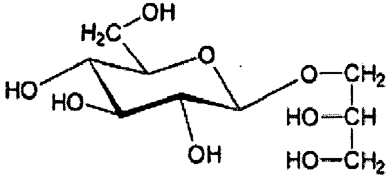 und/oder der allgemeinen Formel 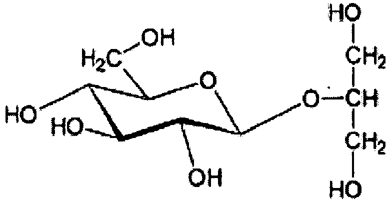 und/oder der allgemeinen Formel 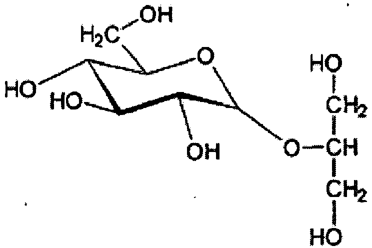 und/oder der allgemeinen Formel 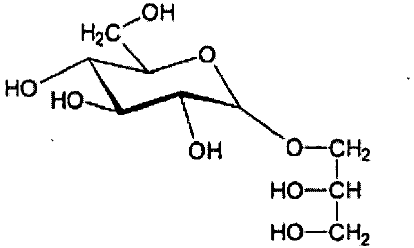 und
(ii) Kreatin und/oder Kreatinin und/oder ein oder mehrere Kreatinderivate gewählt aus der Gruppe Kreatinphosphat, Kreatinsulfat, Kreatinacetat, Kreatinascorbat und der an der Carboxylgruppe mit mono- oder polyfunktionalen Alkoholen veresterten Kreatinederivate, bzw. kosmetische Zubereitungen, solche Wirkstoffkombinationen enthaltend, die Nachteile des Standes der Technik beseitigen.

Es war für den Fachmann daher nicht vorauszusehen gewesen, daß die erfindungsgemäßen Wirkstoffkombinationen bzw. kosmetische oder dermatologische Zubereitungen, diese enthaltend
- besser als feuchtigkeitsspendendes Agens,
- besser gegen die Hautalterung wirken
   besser in Form einer Prophylaxe
- von defizitären, sensitiven oder hypoaktiven Hautzuständen oder defizitären, sensitiven oder hypoaktiven Zustände von Hautanhangsgebilden
- von bestimmten degenerativen Erscheinungen der Haut (z.B. Hauterschlaffung, Altersflecken, Teleangiektasien sowie Schwund der epidermalen und dermalen Zellschichten, der Bestandteile des Bindegewebes, der Retezapfen und Kapillargefässe der Haut) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde.
- von lichtbedingten Hautschäden
- von Pigmentierungsstörungen,
- von Juckreiz,
- von Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo
wirken würde als die Wirkstoffe, Wirkstoffkombinationen und Zubereitungen des Standes der Technik.

Besonders bevorzugtes Glucosylglycerid ist das (2-O-β-D-Glucopyranosyl)-sn-glycerin.

Ein besonders vorteilhafter Citronensäureester ist das Glycerylstearatcitrat. Solche Citronensäureester sind beispielsweise erhältlich unter der Produktbezeichnung "IMWITOR® 370" der Gesellschaft Hüls AG.

Es ist erfindungsgemäß vorteilhaft, das molare Verhältnis von einem oder mehreren Glucosylglyceriden zu Kreatin und/oder Kreatinin und/oder ein oder mehrere Kreatinderivate gewählt aus der Gruppe Kreatinphosphat, Kreatinsulfat, Kreatinacetat, Kreatinascorbat und der an der Carboxylgruppe mit mono- oder polyfunktionalen Alkoholen veresterten Kreatinederivate, aus dem Bereich von 100 : 1 bis 1 : 100 , bevorzugt 50 : 1 bis 1 : 50, insbesondere bevorzugt 20 : 1 bis 1 : 20 zu wählen.

Entsprechend der erfindungsgemäßen Verwendung sind die Zubereitungen besonders vorteilhaft dadurch gekennzeichnet, daß Kreatin und/oder Kreatinin und/oder ein oder mehrere Kreatinderivate gewählt aus der Gruppe Kreatinphosphat, Kreatinsulfat, Kreatinacetat, Kreatinascorbat und der an der Carboxylgruppe mit mono- oder polyfunktionalen Alkoholen veresterten Kreatinderivate, in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Entsprechend der erfindungsgemäßen Verwendung sind die Zubereitungen besonders vorteilhaft dadurch gekennzeichnet, daß das oder die Glucosylglyceriden in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegen.

Entsprechend der erfindungsgemäßen Verwendung können die kosmetischen Desodorantien in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Cremes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der desodorierenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen

Öle, wie Triglyceride der Caprin- oder der Caprylsäure, vorzugsweise aber Rizinusöl;

Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es folgen vorteilhafte Ausführungsbeispiele der vorliegenden Erfindung.

### Beispiel 1:

### Gelcrème

| | Gew.-% |
|---|---|
| Acrylat /C₁₀₋₃₀ Alkylacrylat Crosspolymer | 0,40 |
| Polyacrylsäure | 0,20 |
| XanthangGummi | 0,10 |
| Cetearylalkohol | 3,00 |
| C₁₂₋₁₅ Alkylbenzoat | 4,00 |
| Caprylic/Capric Triglycerid | 3,00 |
| Cyclisches Dimethylpolysiloxan | 5,00 |
| Dimeticon Polydimethylsiloxan | 1,00 |
| Glucosylglycerid | 4,00 |
| Kreatin | 0,50 |
| Kreatinin | 0,001 |
| Glycerin | 3,00 |
| Natriumhydroxid | q.s. |
| Konservierungsmittel | q.s. |
| Parfum | q.s. |
| Wasser | ad 100,0 |
| pH-Wert eingestellt auf 6.0 | |

Durch Mischen der in der Tabelle angegebenen Komponenten wurde eine Gelcreme der ebenfalls angegebenen Zusammensetzung hergestellt. Der pH-Wert der Gelcreme wurde anschließend auf 6,0 eingestellt.

### Beispiel 2:

### W/O-Creme

| | Gew.-% |
|---|---|
| Polyglyceryl-3-Diisostearat | 3,50 |
| Glycerin | 3,00 |
| Polyglyceryl-2-Dipolyhydroxystearat | 3,50 |
| Glucosylglycerid | 4,00 |
| Kreatin | 0,60 |
| Kreatinin | 0,01 |
| Konservierungsmittel | q.s. |
| Parfum | q.s. |
| Magnesiumsulfat | 0,6 |
| Isopropylstearat | 2,0 |
| Caprylylether | 8,0 |
| Cetearylisononanoat | 6,0 |
| Wasser | ad 100,0 |

Durch Mischen der in der Tabelle angegebenen Komponenten wurde eine Creme der ebenfalls angegebenen Zusammensetzung auf der Basis einer Wasser-in-Öl-Dispersion hergestellt.

## Patentansprüche

1. Wirkstoffkombinationen aus
(i) einem oder mehreren Glucosylglyceriden, gewählt aus den Substanzen der allgemeinen Formel 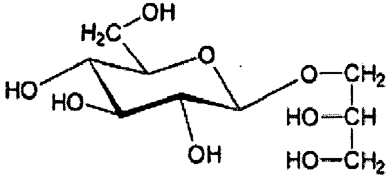 und/oder der allgemeinen Formel 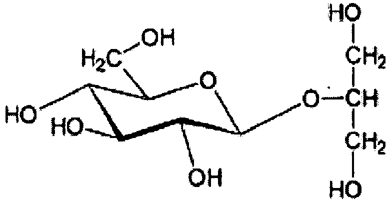 und/oder der allgemeinen Formel 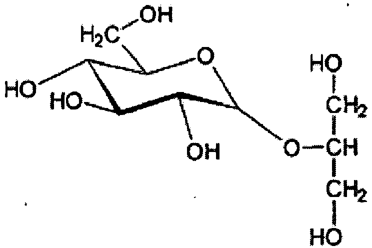 und/oder der allgemeinen Formel 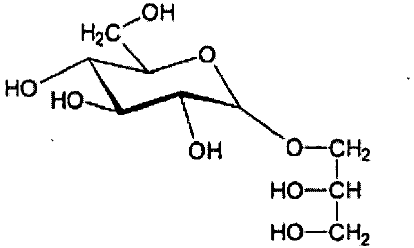
(ii) Kreatin und/oder Kreatinin und/oder einem oder mehrerem Kreatinderivatem gewählt aus der Gruppe Kreatinphosphat, Kreatinsulfat, Kreatinacetat, Kreatinascorbat und der an der Carboxylgruppe mit mono- oder polyfunktionalen Alkoholen veresterten Kreatinderivate.

2. Wirkstoffkombinationen nach Anspruch 1, in denen das molare Verhältnis von einem oder mehreren Glucosylglyceriden zu Kreatin und/oder Kreatinin und/oder ein oder mehrere Kreatinderivate gewählt aus der Gruppe Kreatinphosphat, Kreatinsulfat, Kreatinacetat, Kreatinascorbat und der an der Carboxylgruppe mit mono- oder polyfunktionalen Alkoholen veresterten Kreatinederivate,aus dem Bereich von 100 : 1 bis 1 : 100 , bevorzugt 50 : 1 bis 1 : 50, insbesondere bevorzugt 20 : 1 bis 1 : 20 gewählt wird.

3. Wirkstoffkombinationen nach Anspruch 1 oder 2, **dadurch gekennzeichnet, daß** als Glucosylglycerid das (2-O-β-D-Glucopyranosyl)-sn-glycerin gewählt wird.

4. Kosmetische Zubereitungen, Wirkstoffkombinationen gemäß einem der Ansprüche 1-3 enthaltend.

5. Kosmetische Zubereitungen gemäß Anspruch 4, in denen ein oder mehrere Glucosylglyceride in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt.

6. Kosmetische Zubereitungen gemäß Anspruch 4, in denen Kreatin und/oder Kreatinin und/oder ein oder mehrere Kreatinderivate gewählt aus der Gruppe Kreatinphosphat, Kreatinsulfat, Kreatinacetat, Kreatinascorbat und der an der Carboxylgruppe mit mono- oder polyfunktionalen Alkoholen veresterten Kreatinederivate, in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen

## Claims

1. Active substance combinations of
(i) one or more glucosylglycerides, selected from among the substances of the general formula 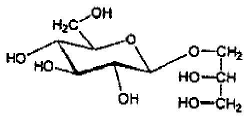 and/or of the general formula 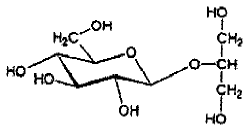 and/or of the general formula 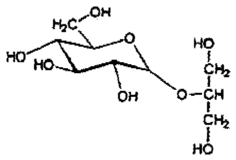 and/or of the general formula 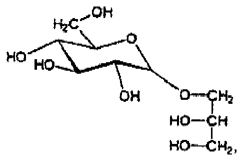
(ii) creatine and/or creatinine and/or one or more creatine derivatives, selected from the group consisting of creatine phosphate, creatine sulphate, creatine acetate, creatine ascorbate and the creatine derivatives which are esterified with monofunctional or polyfunctional alcohols on the carboxyl group.

2. Active substance combinations according to Claim 1, in which the molar ratio of one or more glucosylglycerides to creatine and/or creatinine and/or one or more creatine derivatives, selected from the group consisting of creatine phosphate, creatine sulphate, creatine acetate, creatine ascorbate and the creatine derivatives which are esterified with monofunctional or polyfunctional alcohols on the carboxyl group, is selected from among the range of from 100:1 to 1:100, preferably 50:1 to 1:50, particularly preferably 20:1 to 1:20.

3. Active substance combinations according to Claim 1 or 2, **characterized in that** the glucosylglyceride chosen is (2-O-β-D-glucopyranosyl)-sn-glycerine.

4. Cosmetic preparations, comprising active substance combinations according to one of Claims 1 - 3.

5. Cosmetic preparations according to Claim 4, in which one or more glucosylglycerides are present in concentrations of from 0.01 - 10.00% by weight, preferably 0.05 - 5.00% by weight, especially preferably 0.1 - 3.00% by weight, in each case based on the total weight of the composition.

6. Cosmetic preparations according to Claim 4, in which creatine and/or creatinine and/or one or more creatine derivatives, selected from the group consisting of creatine phosphate, creatine sulphate, creatine acetate, creatine ascorbate and the creatine derivatives which are esterified with monofunctional or polyfunctional alcohols on the carboxyl group, is/are present in concentrations of from 0.01 - 10.00% by weight, preferably 0.05 - 5.00% by weight, especially preferably 0.1 - 3.00% by weight, in each case based on the total weight of the composition

## Revendications

1. Association de substances actives à base
(i) d'un ou plusieurs glucosylglycérides choisis parmi les substances de formule générale 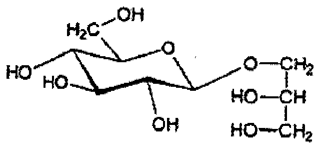 et/ou de la formule générale 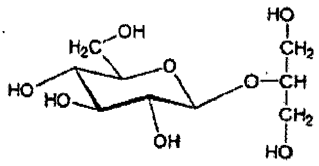 et/ou de la formule générale 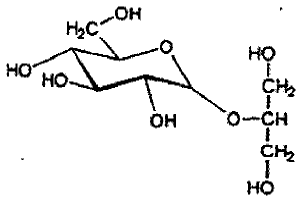 et/ou de la formule générale 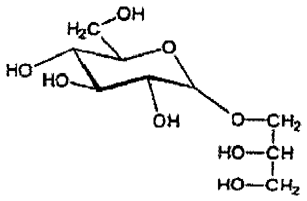
(ii) de créatine et/ou de créatinine et/ou d'un ou plusieurs dérivés de créatine choisis dans le groupe constitué par le phosphate de créatine, le sulfate de créatine, l'acétate de créatine, l'ascorbate de créatine et parmi les dérivés de créatine estérifiés sur le groupe carboxy par des alcools mono- ou polyfonctionnels.

2. Associations de substances actives selon la revendication 1, dans lesquelles le rapport molaire d'un ou plusieurs glucosylglycérides à la créatine et/ou la créatinine et/ou un ou plusieurs dérivés de créatine choisis dans le groupe constitué par le phosphate de créatine, le sulfate de créatine, l'acétate de créatine, l'ascorbate de créatine et les dérivés de créatine estérifiés sur le groupe carboxy par des alcools mono- ou polyfonctionnels, est choisi dans la plage allant de 100 : 1 à 1 : 100, de préférence de 50 : 1 à 1 : 50, de façon particulièrement préférée de 20 : 1 à 1 : 20.

3. Associations de substances actives selon la revendication 1 ou 2, **caractérisées en ce qu'**en tant que glucosylglycéride on choisit le (2-O-β-D-glucopyranosyl)-sn-glycérol.

4. Préparations cosmétiques, contenant des associations de substances actives selon l'une quelconque des revendications 1 - 3.

5. Préparations cosmétiques selon la revendication 4, dans lesquelles un ou plusieurs glucosylglycérides est/sont présent(s) à des concentrations de 0,01 - 10,00 % en poids, de préférence de 0,05 - 5,00 % en poids, de façon particulièrement préférée de 0,1 -3,ou % en poids, chaque fois par rapport au poids total de la composition.

6. Préparations cosmétiques selon la revendication 4, dans lesquelles la créatine et/ou la créatinine et/ou un ou plusieurs dérivés de créatine choisis dans le groupe constitué par le phosphate de créatine, le sulfate de créatine, l'acétate de créatine, l'ascorbate de créatine et les dérivés de créatine estérifiés sur le groupe carboxy par des alcools mono- ou polyfonctionnels est ou sont présent(s) à des concentrations de 0,01 - 10,00 % en poids, de préférence de 0,05 - 5,00 % en poids, de façon particulièrement préférée de 0,1 - 3,00 % en poids, chaque fois par rapport au poids total de la composition.
